Europäisches Patentamt

European Patent Office

Office européen des brevets

(19)

(11) **EP 0 516 617 B1**

(12) **EUROPEAN PATENT SPECIFICATION**

(45) Date of publication and mention
of the grant of the patent:
**21.01.1998 Bulletin 1998/04**

(21) Application number: **90906708.4**

(22) Date of filing: **23.04.1990**

(51) Int Cl.$^6$: **G08C 19/02**, G08C 19/04,
G08C 19/16, H03L 7/00,
A61N 1/36

(86) International application number:
**PCT/US90/01815**

(87) International publication number:
**WO 91/16696 (31.10.1991 Gazette 1991/25)**

(54) **HIGH SPEED REFLECTED IMPEDANCE TELEMETRY SYSTEM FOR IMPLANTABLE DEVICE**

HOCHGESCHWINDIGKEITS-TELEMETRIESYSTEM MIT REFLEKTIERTER IMPEDANZ FÜR
IMPLANTIERBARE VORRICHTUNG

SYSTEME DE TELEMETRIE A GRANDE VITESSE A IMPEDANCE REFLECHIE POUR DISPOSITIF
IMPLANTE

(84) Designated Contracting States:
**DE FR GB IT NL SE**

(43) Date of publication of application:
**09.12.1992 Bulletin 1992/50**

(73) Proprietor: **PACESETTER, INC.**
**Sylmar, CA 91392-9221 (US)**

(72) Inventor: **SILVIAN, Sergiu**
**La Crescenta, CA 91214 (US)**

(74) Representative: **Rees, David Christopher et al**
**Kilburn & Strode**
**30 John Street**
**London WC1N 2DD (GB)**

(56) References cited:
**US-A- 2 567 286      US-A- 4 083 237**
**US-A- 4 223 679      US-A- 4 453 162**
**US-A- 4 532 932      US-A- 4 681 111**
**US-A- 4 847 617**

## Description

## Background of the Invention

Field of the Invention - The present invention relates generally to a telemetry system for use in medical devices of various types and for various purposes implantable within the human body, and more particularly to a telemetry system which will transmit data at a relatively high rate while retaining a high degree of accuracy.

With the ever-shrinking size of electronic circuitry, the implantation into the human body of electronic medical devices has become more and more common. Although the most commonly known of such devices is the cardiac pacemaker, there are a variety of devices which are implanted into the human body, including devices for the stimulation or sensing, or both, of the brain, nerves, the spinal cord, muscles, bones, glands, or other body organs or tissues.

It will be appreciated by those skilled in the art that such implantable devices are becoming increasingly complex, and that more functions can be crammed into a relatively smaller electronic chip or circuit. Implantable medical devices have used bidirectional telemetry for a number of years. Information transmitted between the implanted device and an external transceiver may include, for example, device identification information, biological data, current operational parameters of the device, technical information regarding proper operation of the device, battery charge condition, revised operational parameters (programming information) for the device, and verification of information transmitted between the implanted device and the external transceiver.

With an ever-increasing amount of data being processed and available within the implantable device, there has been an corresponding increase in the need to transmit more data from the implanted device to the external transceiver for analysis, reprogramming of the implantable device, or other purposes. The need for more data to be transmitted in both directions has, increased tremendously the amount of time required both to interrogate the implanted device and to reprogram the implanted device. An upper limit on the amount of data flowing between the implanted device and the external transceiver has therefore become directly proportional to the amount of time which may reasonably be taken to interrogate and reprogram the implanted device.

Accordingly, it is desirable to achieve a higher rate of data transfer between the implanted device and the external transceiver to eliminate this artificial impediment and to maximize the communication between the implanted device and the external transceiver. Of course, an increase in the rate of data exchange may not be obtained at the expense of accuracy in a medical device, particularly if the device is a life-sustaining device such as a pacemaker. Absolute accuracy is required, and it is apparent that an increase in the rate of data transfer will be nullified by an increase in the amount of time spent to verify data to ensure the degree of accuracy required.

The recent development of LSI circuits incorporating low current analog-to-digital converters has made the use of such converters in implantable devices possible. There are, of course, limitations surrounding the design of new implantable systems or portions thereof, the most limiting of which is the consumption of energy by the system. Implanted systems are customarily powered by a long-lasting non-replaceable internal battery, and the current consumption of a telemetry sub-system thus becomes perhaps the most important design factor to be considered.

Previously known devices have established various methods of communicating non-invasively through the skin. For example, U.S. Patent No. 4,223,679, to Schulman et al., which patent is assigned to the assignee of the present invention, shows an implanted device which uses little or no current to transmit information by relying on reflected impedance of an internal L-C circuit. Internal modulation circuits in the implanted device transmit digital or analog data by modulating the reflected impedance, and the external transceiver utilizes an oscillator having varying frequency and amplitude outputs determined by a coupled RF magnetic field carrier to an L-C circuit in the external transceiver. This system works well, but, unfortunately, has speed limitations making it unsuitable for transmitting the amount of information contemplated herein.

Another type of device uses an active type transmitter, with the transmitted energy being taken from the implantable device battery. This type of device, which is illustrated in U.S. Patent No. 4,281,664, to Duggan, has a data rate which is limited to approximately 100 BPS (bits per second). Another device which utilizes an active type transmitter is shown in U.S. Patent No. 4,453,162, to Daly et al. The Daly et al. device can not achieve more than 0.25 to 0.05 bps/carrier cycle, a rate too low for the applications contemplated herein.

Still another telemetry system is disclosed in my U.S. Patent No. 4,681,111, which increases the speed of the device disclosed in above-referenced U.S. Patent No. 4,223,679. However, the maximum rate of this device is still limited by the bandwidth of the external device L-C oscillator. Those skilled in the art will also appreciate that it is not practical to increase the carrier frequency above approximately eight kHz, since the metal enclosure of the implanted device will experience eddy currents great enough to attenuate the signal significantly. In addition, increasing the carrier frequency makes electromagnetic interference from video display terminals, which are generally approximately 16 kHz, a significant problem. Consequently, techniques requiring a number of carrier cycles for each signal bit will be capable of achieving a speed of only two to four kBPS.

One possible solution is disclosed in my U.S. Patent Application No. 07/085,914. This system utilizes both in-phase and quadrature data components, and frequency modulates both data components into a single transmitted sinusoidal signal which varies in frequency between two selected frequencies. The signal is received and decoded, preferably by a coherent decoder, into in-phase and quadrature components, which are then integrated and sampled to produce the two transmitted in-phase and quadrature data components, which may then be recombined to produce the transmitted data. The system requires only low power, and is capable of operating at a relatively high data rate while retaining a high degree of accuracy due to the splitting of the signal into the in-phase and quadrature data components.

It will, however, be appreciated that there exists a substantial need for other telemetry systems which are capable of accurately transmitting and receiving data at a rate enabling the substantial amounts of data used by current implantable systems to be conveniently sent in a relatively short period of time. The amount of power required by the implanted portion of such a system must be minimal, so as not to adversely affect battery life. The system should be compact so as to not add significantly to the space required by the implanted device. Finally, it is also an objective that all of the aforesaid advantages and objectives be achieved without incurring any substantial relative disadvantage.

## Summary of the Invention

The disadvantages and limitations of the background art discussed above are overcome by the present invention, which is defined according to the appended Claims. With this invention, a servo loop is added to a conventional reflected impedance receiving front end circuit using an AM demodulator. Such a circuit typically has an external inductor in parallel with a capacitor to tune the inductor to an implanted inductor from which information is being transmitted. The AM demodulator is connected across the parallel LC combination. An oscillator is used to drive the LC circuit at a desired frequency.

The present invention adds a servo loop between the output of the AM demodulator and the oscillator. The feedback loop compares the voltage output from the AM demodulator with a reference voltage, and thus operates to keep the voltage across the LC circuit constant over time. The feedback loop does not affect short term variations in the voltage across the LC circuit which are caused by the variations in the reflected impedance since the data transmission frequency possible with the system of the present invention is relatively high.

The frequency response of the system of the present invention is thus tailored to the desired response required by a high speed telemetry channel. Mainly, the system frequency response (the response to the implanted device modulation), specified as center frequency and bandwidth, can be specified independently of the L-C oscillator parameters through use of the servo loop. While frequency response of the system without the loop declines from a maximum value at zero frequency, the frequency response of the system of the present invention with the loop is shifted (to be centered around a center frequency) which in turn will permit transmission at a substantially higher data rate.

An amplifier and bandpass filter is driven by the output of the AM demodulator, with the output from the amplifier and bandpass filter driving a decoder. Alternately, an FM demodulator may be used in addition to the AM demodulator to demodulate the signal before it is supplied to the amplifier and bandpass filter and the decoder. The AM demodulator, however, remains in the circuit and is still used as an integral part of the servo loop.

Thus, the system of the present invention is capable of accurately transmitting and receiving data at a high rate. The amount of power required by the implanted portion of such a system is based on the energy provided by the external device and the implanted device has to provide only minimal power, and as such the system of the present invention does not adversely affect battery life. The system is compact so as to not add significantly to the space required by the implanted device. Finally, all of the aforesaid advantages and objectives are achieved without incurring any substantial relative disadvantage.

## Description of the Drawings

These and other advantages of the present invention are best understood with reference to the drawings, in which:

Figure 1 is a functional schematic of a known reflected impedance telemetry system used with an AM demodulator in the receiving end;
Figure 2 illustrates the baseband equivalent to the circuit shown in Figure 1;
Figure 3 shows the frequency response limitations of the system shown in Figure 1;
Figure 4 is a functional schematic of a high speed reflected impedance telemetry system constructed according to the teachings of the present invention;
Figure 5 illustrates the high speed front end of the receiver of the telemetry system shown in Figure 4, with an AM demodulator being used in conjunction with a closed servo loop feedback circuit driving the oscillator;
Figure 6 illustrates the bandpass equivalent to the circuit shown in Figure 5.
Figure 7 illustrates the baseband equivalent to the circuit shown in Figure 6;
Figure 8 shows the system frequency response of the circuit shown in Figure 5;
Figure 9 shows selected signals in the transmitter and receiver; and
Figure 10 is a functional schematic of a high speed reflected impedance telemetry system similar to that shown in Figure 4, but with an FM demodulator being used in addition.

## Detailed Description of the Preferred Embodiment

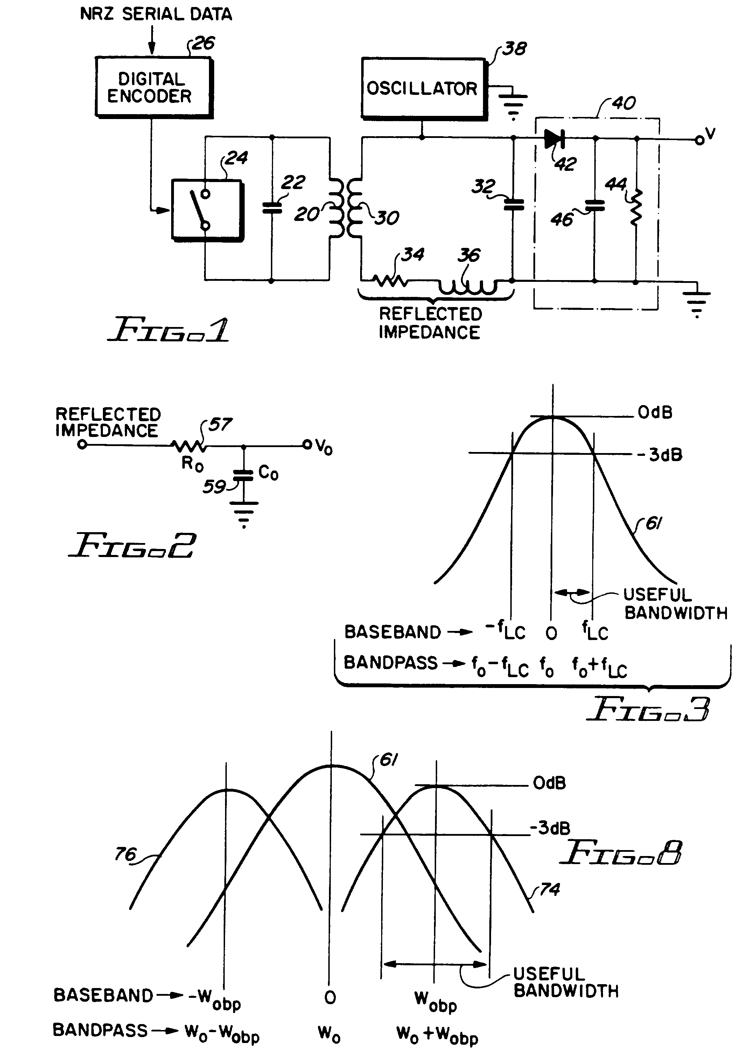

Before discussing the preferred embodiment of the present invention, it is useful to examine a known reflected impedance telemetry system to determine its shortcomings. Such a system is illustrated in Figure 1, which shows both the implanted and the external portions of such a system. An inductor or coil 20 is tuned to a resonant frequency $f_0$ by a capacitor 22 which is connected in parallel across the inductor 20. Also connected in parallel across the inductor 20 and the capacitor 22 is a low impedance semiconductor switch 24, which is typically a CMOS device.

The external receiver used to receive data transmitted from the implanted device has a coil or inductor 30 which is applied to the surface of the skin over the location of the inductor 20 of the implanted device. The two inductors 20 and 30 are constructed in a manner whereby they will magnetically couple to each other. As such, they may be regarded as two coils of a transformer having a low coupling coefficient. A capacitor 32 is connected across the inductor 30.

With the switch 24 open, the circuit will oscillate at the frequency $f_0$ in an essentially sinusoidal manner, with a sinusoidal voltage $V_L$ across the inductor 20 and the capacitor 22. In this state, the circuit will dissipate energy taken from the external tank circuit including the inductor 30 and the capacitor 32. When the switch 24 is closed, the inductor 20 and the capacitor 22 are shorted. In this state, the circuit will dissipate lower energy taken from the external tank circuit including the inductor 30 and the capacitor 32. Accordingly, by opening and closing the switch 24, the voltage on the capacitor 32 is AM and FM modulated.

Non-return-to-zero (NRZ) serial binary data is supplied by the implanted device to a digital encoder 26, which is used to open and close the switch 24 to modulate the voltage signal across the inductor 20. In the example used herein, an NRZ data bit of "0" is encoded by the digital encoder 26 to be two excursions from zero to one or from one to zero, while an NRZ data bit of "1" is encoded by the digital encoder 26 to be a single excursion from zero to one or from one to zero (see Figure 9).

A reflected impedance from the implanted circuit appears in series with the inductor 30 and the capacitor 32. The reflected impedance is illustrated in Figure 1 by a resistor 34 and an inductor 36 connected in series together with the inductor 30 and the capacitor 32. When the switch 24 is open, the resistance of the resistor 34 is high, and when the switch 24 is closed, the resistance of the resistor 34 is very low, near zero. When the switch 24 is opened, the inductance of the inductor 36 will have a low negative value, and while the switch 24 is closed the inductor 36 will have approximately zero inductance.

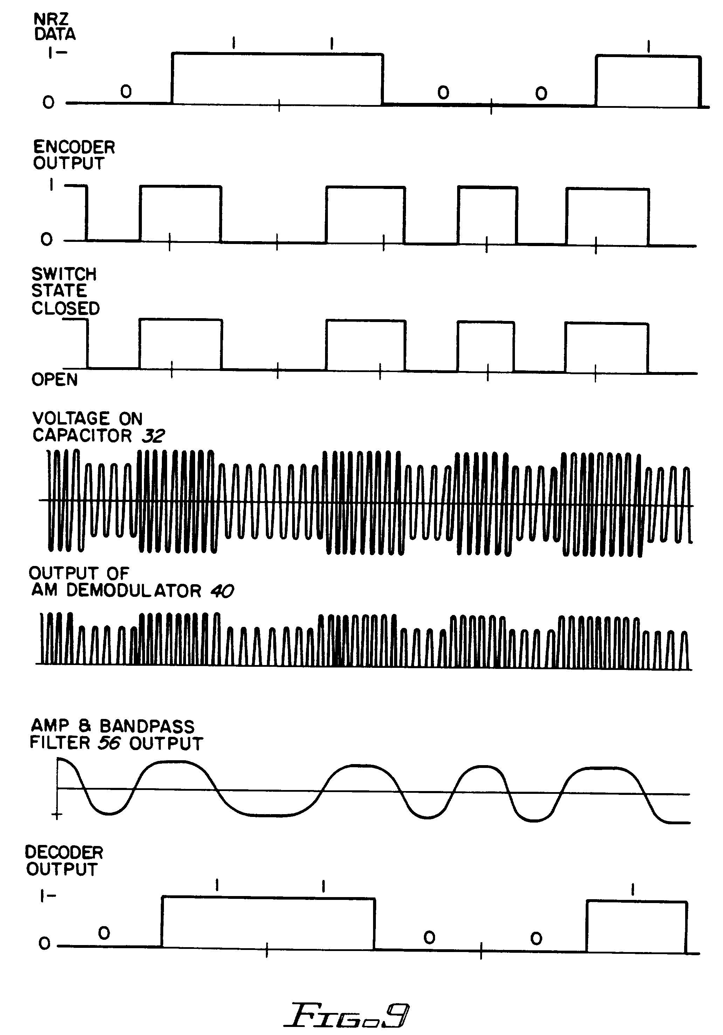

An oscillator 38 is connected to the tuned external circuit, and oscillates approximately at $f_0$. As such, the inductor 30 and the capacitor 32 will oscillate at a frequency which is approximately $f_0$. Generally, the inductor 30 and the capacitor 32 are tuned to $f_0$ to accomplish an efficient transfer of energy. The oscillator 38 may be a Colpitts circuit, or the circuit shown in Figure 2 in U.S. Patent No. 4,233,679, to Schulman.

As the switch 24 is opened and closed, the voltage across the capacitor 32 will change, as will the frequency of oscillation. When the switch 24 is opened, the voltage across the capacitor 32 will drop due to an increase in reflected resistance, and the frequency of oscillation will increase slightly. Therefore, it is apparent that as the switch 24 is opened and closed, the voltage across the capacitor 32 will be modulated. In addition, the main frequency of oscillation of the circuit in the external device, which is $f_0$, will be modulated somewhat by the switch 24 being opened and closed.

It will therefore be appreciated that the modulation imposed by the switch 24 on the implanted tuned circuit will appear across the capacitor 32. Accordingly, a demodulator may be used to obtain the data being transmitted. In the circuit shown in Figure 1, an AM demodulator 40 is used. The AM demodulator 40 will extract the AM modulation produced by the reflected impedance, with the output $V_o$ of the AM demodulator 40 containing the DC oscillator amplitude plus the AC modulation.

The AM demodulator 40 may typically be a diode 42 having its anode connected to the ungrounded side of the capacitor 32, with its cathode being the output of the circuit at which $V_o$ appears. A resistor 44 and a capacitor 46 are connected together in parallel between the cathode of the diode 42 and ground. It will of course be recognized by those skilled in the art that there are various other AM demodulator designs, and any of them could alternatively be used.

Typically an amplifier and band pass filter (not shown) may then be used to filter out the carrier frequency, thus extracting, amplifying and band limiting the AC signal. Thus, only the frequency portion of the signal necessary to reconstruct the transmitted data is passed on. A decoder (not shown), is then used to decode the signal to provide the original transmitted data.

The most important characteristic of the system shown in Figure 1 is its maximum data rate. Consider for the moment that the digital encoder 26 is removed from the circuit of Figure 2, and that the NRZ serial data is applied directly to the switch 24. The AM modulation used is typically a double sideband type, since both sidebands are transmitted, and the frequency spectrum of the NRZ serial data itself is infinite. For a binary communication to be placed, the system bandwidth must be larger than the minimum Nyquist rate of $\frac{1}{2T} = 0.5 f_r$, where $T$ is the bit duration and $f_r$ is the bit rate.

From practical considerations, generally the bandwidth is not smaller than $0.75 f_r$. A double sideband AM modulation will thus require at least a $1.5 f_r$ bandwidth.

The oscillator 38, the inductor 30, and the capacitor 32 are equivalent to a 2-pole bandpass filter which passes the AM modulation on the $f_o$ carrier. Figure 2 illustrates the baseband equivalent to the circuit of Figure 1. In Figure 1, a resistor 57 has one side as the input to the circuit, with the input being the reflected impedance. The other side of the resistor 57 is the output of the circuit on which $V_o$ appears. Connected across the output of the circuit and ground is a capacitor 59.

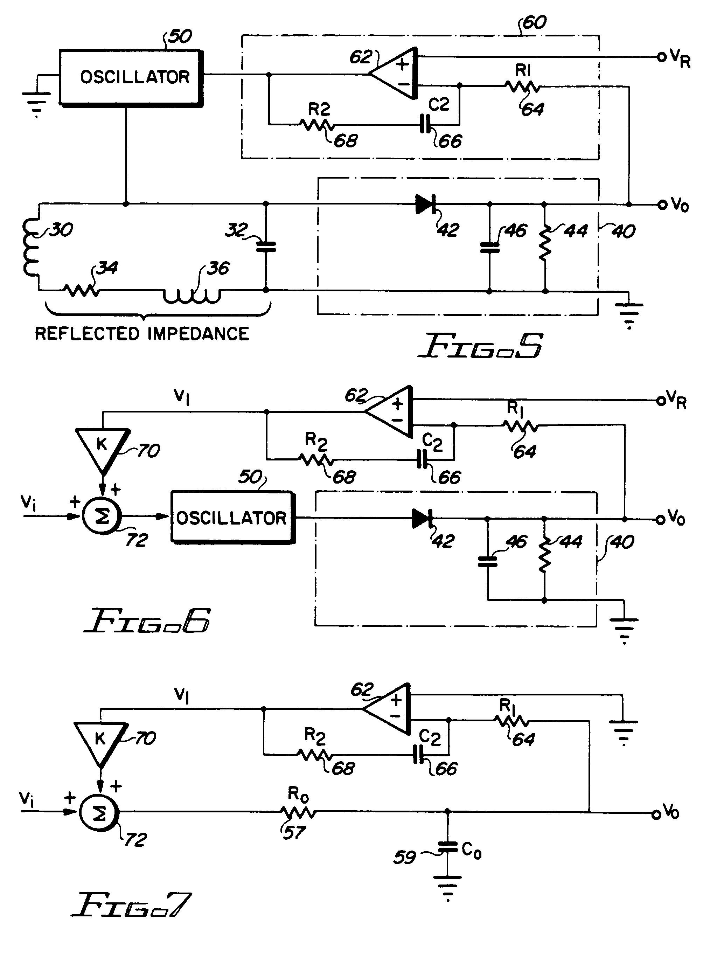

Figure 3 shows the frequency response of the system shown in Figure 1 (which response is indicated by the reference numeral 61). At baseband (after AM demodulation), and assuming the AM demodulator to be perfect with no modulation frequency alteration, the system is equivalent to a one-pole lowpass filter having a bandwidth $f_{LC}$. The width of this bandwidth $f_{LC}$ is limited by the coil quality of factor Q as defined by the formula

$$(1) \qquad f_{LC} = \frac{1}{2} \frac{f_0}{Q} = \frac{1}{2\pi R_0 C_0}.$$

In practice, the maximum rate at which transmitted data may be accurately received will be $f_{LC}$, since at frequencies above $f_{LC}$ the amplitude of the signal will have dropped more than 3 dB. Accordingly, the maximum rate of transmission of data will be $f_{LC}$, which for the system shown in Figure 1 is only approximately 400 Hz. This effectively limits the rate of data transmission to a relatively low rate.

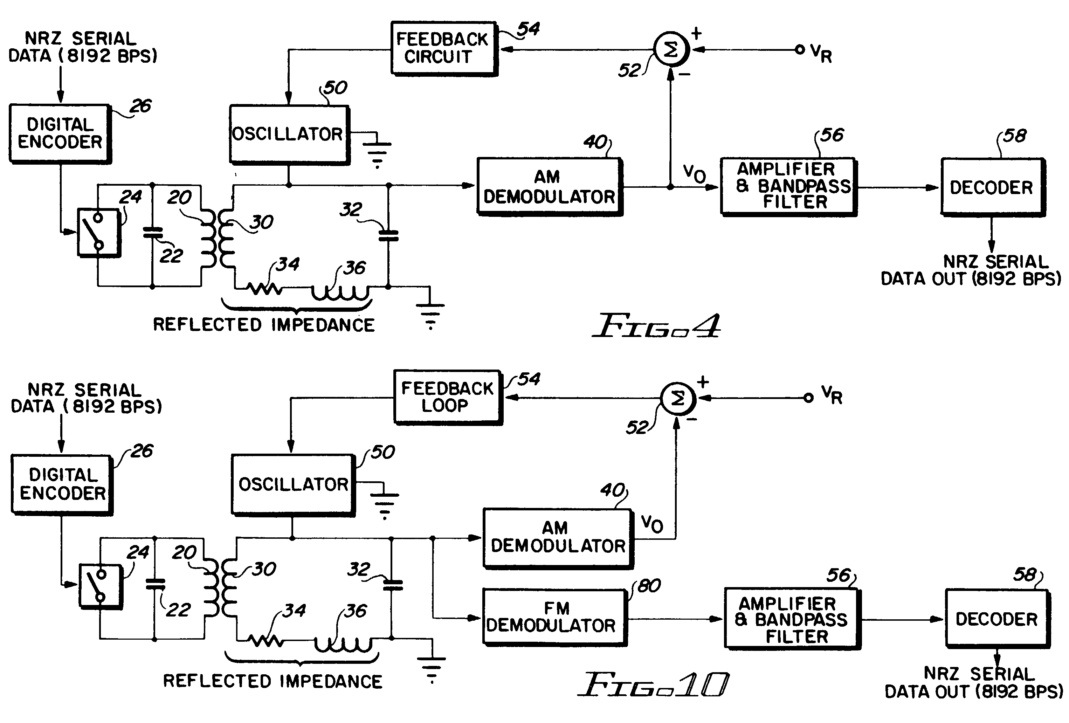

Referring now to Figure 4, a system illustrating the preferred embodiment of the present invention is shown. The implanted portion of the system is identical to the system of Figure 1, and there are other similarities in the external portion of the system. The system of Figure 4 uses the inductor 30 which is applied to the surface of the skin over the location of the inductor 20 of the implanted device to couple the two inductors 20 and 30 magnetically to each other. The capacitor 32 is connected across the inductor 30, with the reflected impedance from the implanted circuit being the resistor 34 and the inductor 36.

The system of Figure 4 also uses an oscillator 50 which is connected to the tuned external circuit, and which will oscillate approximately at $f_0$. The inductor 30 and the capacitor 32 are again tuned to $f_0$ to accomplish an efficient transfer of energy. Again, as the switch 24 is opened and closed, the voltage and frequency across the capacitor 32 will be modulated.

Accordingly, a demodulator may be used to obtain the data being transmitted, and the AM demodulator 40 is again used. The AM demodulator 40 will extract the AM modulation produced by the reflected impedance, with the output $V_o$ of the AM demodulator 40 containing the DC oscillator amplitude plus the AC modulation. The circuit of Figure 4 differs from the circuit of Figure 1 in that a feedback loop is used between the output $V_o$ of the AM demodulator 40 and the oscillator 50 which is used to drive the external circuit.

A comparator 52 is used to compare the AM demodulator 40 output $V_o$ with a reference voltage $V_R$. The output of the comparator 52 is supplied to a feedback circuit 54, the output of which is used to drive the oscillator 50. The feedback loop will thus operate to keep the voltage across the capacitor 32 constant over time. It must be noted at this point that the feedback loop will not affect short term variations in the voltage across the capacitor 32 which are caused by the variations in the reflected impedance caused by the switch 24 being opened and closed. This is so since the frequency at which the switch 24 is opened and closed is relatively high, as will become evident below.

Also connected to the output of the AM demodulator 40 is an amplifier and bandpass filter 56, which is used to amplify and filter the signal from the AM demodulator 40. Finally, a decoder 58 is used to decode the amplified and filtered signal to obtain the original data. The amplifier and bandpass filter 56 and the decoder 58 are of standard design and are well known in the art.

Referring next to Figure 5, a circuit 60 which will perform the functions of the comparator 52 and the feedback circuit 54 is shown. In this circuit 60, an operational amplifier 62 is shown which is connected as an integrator. The reference voltage $V_R$ is connected to the positive input of the operational amplifier 62. A resistor 64 is connected between the output $V_o$ of the AM demodulator 40 and the negative input of the operational amplifier 62. A capacitor 66 is connected on one side to the negative input of the operational amplifier 62, and on the other side to one side of a resistor 68. The other side of the resistor 68 is connected to the output of the operational amplifier 62.

To complete the feedback loop, the output of the operational amplifier 62 is connected to the oscillator 50. The output from the operational amplifier 62 will keep the voltage output from the oscillator 50 constant over time, as mentioned above. This feedback loop has two essential effects. The first of these effects is that the amplitude of the output from the oscillator 50 will be such that the DC output from the AM detector 40 will be constant, equal to $V_R$.

The second effect of the feedback loop is that the transfer function from the reflected impedance modulation to the output $V_o$ of the AM demodulator 40 will no longer be a 1-pole lowpass response, but rather a bandpass response. The bandpass equivalent of the circuit shown in Figure 5 is shown in Figure 6. The output from the operational amplifier 62 is connected to drive an amplifier 70. A coefficient K is equal to the derivative of $V_o$ divided by the derivative of $V_1$. The output of the amplifier 70 is supplied as an input to a summer 72, the other input of which is the external modulation $V_i$ caused by the variations in the reflected impedance. The output of the summer 72 drives the oscillator 50.

Taking the lowpass baseband equivalent of the circuit shown in Figure 6, we obtain the circuit of Figure 7.

The positive input of the operational amplifier 62 is grounded. Accordingly, the output of the summer 72 is supplied to the one side of the resistor 57, with the other side of the resistor 57 being the output $V_o$. The capacitor 59 is connected across the output $V_o$ and ground. The resistor 57 and the capacitor 59 make up a four pole low pass filter equivalent to the one shown in Figure 2.

By performing the following elementary control loop calculations, the determination of an overall input-output transfer function H(s) may be made:

$$(2) \qquad H(s) = \frac{V_o}{V_i}$$

$$(3) \qquad \omega_{LC} = 2\pi f_{LC} = \frac{1}{R_o C_o}$$

$$(4) \qquad H_{LC} = \frac{1}{1 + \dfrac{s}{\omega_{LC}}}$$

$$(5) \qquad \frac{V_o}{V_i} = \frac{H_{LC}}{1 - H_{CL}H_f} = H(s)$$

$$(6) \qquad H_f = -K \frac{1 + R_2 C_2 s}{R_1 C_2 s}$$

$$(7) \qquad H(s) = \frac{\omega_{LC} s}{s^2 + \dfrac{R_1 + KR_2}{R_1} \omega_{LC} s + K \dfrac{\omega_{LC}}{R_1 C_2}}$$

$$(8) \qquad \omega_{obp} = \sqrt{K \frac{\omega_{LC}}{R_1 C_2}}$$

$$(9) \qquad Q = \frac{R_1}{R_1 + KR_2} \frac{\omega_{obp}}{\omega_{LC}}$$

By inspection, it may be seen that H(s) is a standard 2-pole bandpass response having a center frequency $W_{obp}$ and a Quality factor Q which may be tailored to the application, and which are no longer solely determined by the oscillator L-C elements. Figure 8 illustrates how the new frequency response (indicated by the reference numerals 74 and 76) appears compared to the L-C response of the system of Figure 1 (indicated by the reference numeral 61, which response was also illustrated in Figure 3).

As may be seen from the H(s) relation (equation 7) above and from Figure 8, the system response 74 of the system shown in Figure 4 at zero frequency is zero as opposed to maximum in the systems of Figure 1. The system response 74 instead is centered around the frequency $\omega_{obp}$, and the useful bandwidth is substantially wider than the useful bandwidth of the system of Figure 1.

Referring for the moment to Figure 4, the digital encoder 26 has the task of changing the frequency spectrum from NRZ (non-return-to-zero), which has components down to DC, into a spectrum which contains no components close to zero frequency. In the preferred embodiment of the present invention, an MSK (minimum-shift-keying) encoder is used which has a first frequency $f_1$ which is 8192 Hz, and a second frequency $f_2$ which is 4096 Hz. In the preferred embodiment, there is a straight coding rule in which a zero will be transmitted as $f_1$ and a 1 will be transmitted as $f_2$, as shown in Figure 9.

The voltage across the capacitor 32 is shown in Figure 9, with the associated output from the AM demodulator 40, which is one side of the voltage across the capacitor 32. The amplifier and bandpass filter 56 produces a signal responsive to the non-DC portion of the envelope of the output from the AM demodulator 40. The signal output of the amplifier and bandpass filter 56 will then be used by the decoder 58 to decode the signal back to NRZ.

Construction of the decoder 58 will be apparent to one skilled in the art. An MSK encoding is known to require a relatively low transmission bandwidth. In fact it can work on a two-sided bandwidth of 0.7 divided by the period T. This results in the factor 0.7 being multiplied by the frequency 8192 Hz to obtain a 5734 Hz bandwidth which is centered on $f_{obp}$ of (8192 + 4096)/2, or 6144 Hz.

The choice of $f_1$ and $f_2$ can lead to simplification of the decoder 58. The best decoder performance will be obtained using a coherent decoder which, using a PLL (phase-locked-loop), regenerates at the receiver the two carriers $f_1$ and $f_2$, as well as the bit clock timing. However, here the bit clock is equal to $f_1$ and $f_2 = 1/2f_1$, so the carriers may be obtained very simply. A non-coherent decoder could also be used, with reduced performance but with simplified circuits. Detailed circuit schematics for the digital encoder 26, the amplifier and bandpass filter 56, and the decoder 58 are not presented since they may be implemented in a multitude of ways well known to persons skilled in the art.

Referring next to Figure 10, is should be noted that while the AM demodulator 40 is necessarily contained in the loop, an FM demodulator 80 could also be connected with its input across the capacitor 32. The output of the FM demodulator 80 would then drive the amplifier and bandpass filter 56.

It should also be noted that such telemetry systems typically have provisions to work in the opposite direction, that is from the external device to the implanted device. In this case, a receiver detector would be connected across the inductor 20.

It may therefore be appreciated from the above detailed description of the preferred embodiment of the present invention that it is capable of accurately transmitting and receiving data at a high rate. The amount of power required by the implanted portion of such a system is minimal, and as such does not adversely affect battery life. The system is compact so as to not add significantly to the space required by the implanted device. Finally, all of the aforesaid advantages and objectives are achieved without incurring any substantial relative disadvantage.

Although an exemplary embodiment of the present invention has been shown and described, it will be apparent to those having ordinary skill in the art that a number of changes, modifications, or alterations to the invention as described herein may be made which fall within the scope of the appended claims defining the present invention. All such changes, modifications, and alterations should therefore be seen as within the scope of the present invention.

**Claims**

1. A telemetry system for use with an implantable device, comprising:

   an inductor for receiving an information-containing magnetic field;
   a capacitor connected in parallel across said inductor;
   an oscillator connected to drive said inductor and said capacitor with an oscillating output signal;
   an AM demodulator connected across said inductor and said capacitor, said AM demodulator having an output containing an AC component and a DC component; said system being characterised by
   feedback loop means connected between the output of said AM demodulator and said oscillator for controlling the amplitude of the oscillating output signal, said feedback loop means for maintaining the DC component of the output of said AM demodulator constant.

2. A telemetry system as defined in Claim 1, wherein said inductor and said capacitor have values to tune them to a frequency $f_0$, and said oscillator is set to oscillate at a frequency which is approximately $f_0$.

3. A telemetry system as defined in Claim 1, wherein said oscillator is controlled by said feedback loop means to maintain the signal driving said inductor and said capacitor at a constant level.

4. A telemetry system as defined in Claim 1, said inductor having a first terminal and a second terminal, said capacitor having a first terminal and a second terminal, said first terminal of said inductor being connected to said first terminal of said capacitor, said second terminal of said inductor being connected to said second terminal of said capacitor, said second terminals being grounded, wherein said AM demodulator comprises:

   a diode having a cathode and an anode, said anode connected to said first terminals of said inductor and said capacitor;
   a second capacitor having first and second terminals, said first terminal of said second capacitor being connected to said cathode of said diode, said second terminal of said second capacitor being connected to said second terminal of said inductor and said second terminal of said capacitor ;
   a resistor having first and second terminals, said first terminal of said resistor being connected to said first terminal of said second capacitor and said cathode of said diode, said second terminal of said resistor being connected to said second terminal of said second capacitor, said second terminal of said inductor, and said second terminal of said capacitor.

5. A telemetry system as defined in Claim 1, wherein said feedback loop means is so arranged and configured to convert the response of said system from a 1-pole lowpass response to a bandpass response.

6. A telemetry system as defined in Claim 1, wherein said feedback loop means comprises:

   an operational amplifier having a positive input, a negative input, and an output, said positive input of said operational amplifier being supplied with a DC reference voltage;
   a first resistor connected between the output of said AM demodulator and said negative input of said operational amplifier;
   a second capacitor having a first terminal and a second terminal, said first terminal of said second capacitor being connected to said negative input of said operational amplifier; and
   a second resistor having a first terminal and a second terminal, said first terminal of said second resistor being connected to said second terminal of said second capacitor, said second terminal of said second resistor being connected to the output of said operational amplifier, the output of said operational amplifier also being connected to drive said oscillator.

7. A telemetry system as defined in Claim 1, additionally comprising:

an amplifier and bandpass filter having as an input the output from said AM demodulator, said amplifier and bandpass filter providing an output; and

a decoder having as an input the output from said amplifier and bandpass filter.

8. A telemetry system as defined in Claim 1, additionally comprising:

an FM demodulator connected across said inductor and said capacitor, said FM demodulator having an output;

an amplifier and bandpass filter having as an input the output from said fM demodulator, said amplifier and bandpass filter providing an output; and

a decoder having as an input the output from said amplifier and bandpass filter.

9. A telemetry system as claimed in any preceding claim further comprising:

a further inductor for transmitting said information-containing magnetic field;

a further capacitor connected in parallel across said first inductor;

a switch connected in parallel across said further inductor and said further capacitor; and

means for periodically, sequentially opening and closing said switch to modulate a voltage across said first inductor and said first capacitor.

10. A telemetry system as defined in Claim 9, wherein said means for periodically, sequentially opening and closing said switch comprises:

a digital encoder, said switch being open when the output of said digital encoder is a digital "0" and closed when the output of said digital encoder is a digital "1."

11. A telemetry system as defined in Claim 10, wherein a data bit of "0" is encoded by said digital encoder to be two excursions from zero to one or from one to zero, while a data bit of "1" is encoded by said digital encoder to be a single excursion from zero to one or from one to zero.

12. A telemetry system as defined in claim 9, wherein said further inductor and said further capacitor have values to tune them to a frequency $f_0$, and said oscillator is set to oscillate at a frequency which is approximately $f_0$.

13. A telemetry system as defined in claim 12, wherein said inductor and said capacitor have values to tune them to said frequency $f_0$.

14. A telemetry system as defined in Claim 9, wherein said oscillator is controlled by said feedback loop means to maintain the signal driving said inductor and said capacitor at a constant level.

15. A telemetry system as defined in Claim 9, wherein said feedback loop means is so arranged and configured to convert the response of said system from a 1-pole lowpass response to a bandpass response.

16. A telemetry system as defined in Claim 9, wherein said feedback loop means comprises:

an operational amplifier having a positive input, a negative input, and an output, said positive input of said operational amplifier being supplied with a DC reference voltage;

a first resistor connected between the output of said AM demodulator and said negative input of said operational amplifier;

a second capacitor having a first terminal and a second terminal, said first terminal of said second capacitor being connected to said negative input of said operational amplifier; and

a second resistor having a first terminal and a second terminal, said first terminal of said second resistor being connected to said second terminal of said second capacitor, said second terminal of said second resistor being connected to the output of said operational amplifier, the output of said operational amplifier also being connected to drive said oscillator.

17. A telemetry system as defined in Claim 9, additionally comprising:

an amplifier and bandpass filter having as an input the output from said AM demodulator, said amplifier and bandpass filter providing an output; and

a decoder having as an input the output from said amplifier and bandpass filter.

18. A telemetry system as defined in Claim 9, additionally comprising:

an FM demodulator connected across said inductor and said capacitor, said FM demodulator having an output;

an amplifier and bandpass filter having as an input the output from said fM demodulator, said amplifier and bandpass filter providing an output; and

a decoder having as an input the output from said amplifier and bandpass filter.

**19.** A method of telemetering data between an implantable device and an external device, comprising:

transmitting an information-containing magnetic field;

receiving an information-containing magnetic field with an inductor;

connecting a capacitor in parallel across said inductor;

driving said inductor and said capacitor with an oscillator having an oscillating output signal;

demodulating the signal across said inductor and said capacitor with an AM demodulator, said AM demodulator having an output containing an AC component and a DC component; said method being characterised by

maintaining the DC component of the output of said AM demodulator constant by connecting feedback loop means between the output of said AM demodulator and said oscillator to control the amplitude of the oscillating output signal.

**Patentansprüche**

1. Telemetriesystem zur Verwendung in einer implantierbaren Vorrichtung, umfassend:

einen Induktor zum Empfangen eines Information enthaltenden magnetischen Feldes;

einen Kondensator, der parallel zu dem Induktor verbunden ist;

einen Oszillator, der angeschlossen ist, um den Induktor und den Kondensator mit einem oszillierenden Ausgangssignal anzutreiben;

einen AM-Demodulator, der parallel zu dem Induktor und dem Kondensator geschaltet ist, wobei der AM-Demodulator einen Ausgang hat, der eine Wechselstromkomponente und eine Gleichstromkomponente enthält, wobei das System **gekennzeichnet ist durch**

Rückkopplungsschleifeneinrichtungen, die zwischen dem Ausgang des AM-Demodulators und dem Oszillator zur Steuerung der Amplitude des oszillierenden Ausgangssignals angeschlossen sind, wobei die Rückkopplungsschleifeneinrichtungen zum konstanten Aufrechterhalten der Gleichspannungskomponente des Ausgangs des AM-Demodulators dienen.

2. Telemetriesystem nach Anspruch 1, worin der Induktor und der Kondensator Werte haben, um sie auf eine Frequenz $f_0$ abzustimmen und der Oszillator festgesetzt wird, um bei einer Frequenz zu oszillieren, welche ungefähr $f_0$ ist.

3. Telemetriesystem nach Anspruch 1, worin der Oszillator durch die Rückkopplungsschleifeneinrichtungen gesteuert wird, um das Signal, welches von dem Induktor und dem Kondensator bei einem konstanten Niveau angetrieben wird, aufrechtzuerhalten.

4. Telemetriesystem nach Anspruch 1, worin der Induktor einen ersten Anschluß und einen zweiten Anschluß hat, wobei der Kondensator einen ersten Anschluß und einen zweiten Anschluß hat, wobei der erste Anschluß des Induktors mit dem ersten Anschluß des Kondensators verbunden ist, der zweite Anschluß des Induktors mit dem zweiten Anschluß des Kondensators verbunden ist, wobei die zweiten Anschlüsse geerdet sind, worin der AM-Demodulator umfaßt:

eine Diode, die eine Kathode und eine Anode hat, wobei die Anode mit den ersten Anschlüssen des Induktors und des Kondensators verbunden ist;

einen zweiten Kondensator, der erste und zweite Anschlüsse hat, wobei der erste Anschluß des zweiten Kondensators mit der Kathode der Diode verbunden ist, wobei der zweite Anschluß des zweiten Kondensators mit dem zweiten Anschluß des Induktors und dem zweiten Anschluß des Kondensators verbunden ist;

einen Widerstand, der erste und zweite Anschlüsse hat, wobei der erste Anschluß des Widerstands mit dem ersten Anschluß des zweiten Kondensators und der Kathode der Diode verbunden ist, wobei der zweite Anschluß des Widerstands mit dem zweiten Anschluß des zweiten Kondensators, dem zweiten Anschluß des Konduktors und dem zweiten Anschluß des Kondensators verbunden ist.

5. Telemetriesystem nach Anspruch 1, worin die Rückkopplungsschleifeneinrichtungen so angeordnet und aufgebaut sind, daß sie die Antwort des Systems von einer I-Pol-Tiefpaßantwort in eine Bandpaßantwort umwandeln.

6. Telemetriesystem nach Anspruch 1, worin die Rückkopplungsschleifeneinrichtungen umfassen:

einen Funktionsverstärker, der einen positiven Eingang, einen negativen Eingang und einen Ausgang hat, wobei der positive Eingang des Funktionsverstärkers mit einer Referenzgleichspannung geliefert wird;

einen ersten Widerstand, der zwischen dem Ausgang des AM-Demodulators und dem negativen Eingang des Funktionsverstärkers angeschlossen ist;

einen zweiten Kondensator, der einen ersten Anschluß und einen zweiten Anschluß hat, wobei der erste Anschluß des zweiten Kondensators mit dem negativen Eingang des Funktionsverstärkers verbunden ist; und

einen zweiten Widerstand, der einen ersten Anschluß und einen zweiten Anschluß hat, wobei der erste Anschluß des zweiten Widerstands mit dem zweiten Anschluß des zweiten Kondensators verbunden ist, wobei der zweite Anschluß des zweiten Widerstands mit dem Ausgang des Funktionsverstärkers verbunden ist, wobei der Ausgang des Funktionsverstärkers ebenso angeschlossen ist, um den Oszillator anzutreiben.

7. Telemetriesystem nach Anspruch 1, zusätzlich umfassend:

einen Verstärker und einen Bandpaßfilter, die den Ausgang von dem AM-Demodulator als einen Eingang haben, wobei der Verstärker und der Bandpaßfilter einen Ausgang liefern; und einen Dekodierer, der den Ausgang von dem Verstärker und dem Bandpaßfilter als einen Eingang hat.

8. Telemetriesystem nach Anspruch 1, zusätzlich umfassend:

einen FM-Demodulator, der parallel zu dem Induktor und dem Kondensator angeschlossen ist, wobei der FM-Demodulator einen Ausgang hat;
einen Verstärker und einen Bandpaßfilter, die als einen Eingang den Ausgang von dem FM-Demodulator haben, wobei der Verstärker und der Bandpaßfilter einen Ausgang liefern; und einen Dekodierer, der als einen Eingang den Ausgang von dem Verstärker und dem Bandpaßfilter hat.

9. Telemetriesystem nach einem der vorangegangenen Ansprüche, weiterhin umfassend:

einen weiteren Induktor zum Senden des Information enthaltenden magnetischen Feldes;
einen weiteren Kondensator, der parallel zu dem ersten Induktor angeschlossen ist;
einen Schalter, der parallel zu dem weiteren Induktor und dem weiteren Kondensator angeschlossen ist;
Einrichtungen zum periodischen sequenziellen Öffnen und Schließen des Schalters, um eine Spannung parallel zu dem ersten Induktor und dem ersten Kondensator zu modulieren.

10. Telemetriesystem nach Anspruch 9, worin die Einrichtungen zum periodischen und sequentiellen Öffnen und Schließen des Schalters umfassen:

einen digitalen Kodierer, wobei der Schalter geöffnet wird, wenn der Ausgang des digitalen Kodierers ein digitales „0" ist und geschlossen wird, wenn der Ausgang des digitalen Kodierers ein digitales "1" ist.

11. Telemetriesystem nach Anspruch 10, worin ein Datenbit von „0" durch den digitalen Kodierer kodiert wird, um zwei Auslenkungen von null zu eins oder von eins zu null zu sein, während ein Datenbit von "1" durch den digitalen Kodierer kodiert wird, um ein einzelne Auslenkung von null auf eins oder von eins auf null zu sein.

12. Telemetriesystem nach Anspruch 9, worin der weitere Induktor und der weitere Kondensator Werte haben, um sie auf eine Frequenz $f_0$ abzustimmen, und der Oszillator gesetzt wird, um bei einer Frequenz zu oszillieren, welche ungefähr $f_0$ ist.

13. Telemetriesystem nach Anspruch 12, worin der Induktor und der Kondensator Werte haben, um sie auf die Frequenz $f_0$ abzustimmen.

14. Telemetriesystem nach Anspruch 9, worin der Oszillator durch die Rückkopplungsschleifeneinrichtungen gesteuert wird, um das Signal, das den Induktor und den Kondensator antreibt, bei einem konstanten Niveau aufrechtzuerhalten.

15. Telemetriesystem nach Anspruch 9, worin die Rückkopplungsschleifeneinrichtungen so angeordnet und aufgebaut sind, daß sie die Antwort des Systems von einer I-Pol-Tiefpaßantwort auf eine Bandpaßantwort umwandeln.

16. Telemetriesystem nach Anspruch 9, worin die Rückkopplungsschleifeneinrichtungen umfassen:

einen Funktionsverstärker, der einen positiven Eingang, einen negativen Eingang und einen Ausgang hat, wobei der positive Eingang des Funktionsverstärkers mit einer Referenzgleichspannung geliefert wird;
einen ersten Widerstand, der zwischen dem Ausgang des AM-Demodulators und dem negativen Eingang des Funktionsverstärkers angeschlossen ist;
einen zweiten Kondensator, der einen ersten Anschluß und einen zweiten Anschluß hat, wobei der erste Anschluß des zweiten Kondensators mit dem negativen Eingang des Funktionsverstärkers verbunden ist; und
einen zweiten Widerstand, der einen ersten Anschluß und einen zweiten Anschluß hat, wobei der erste Anschluß des zweiten Widerstands mit dem zweiten Anschluß des zweiten Kondensators verbunden ist, der zweite Anschluß des zweiten Widerstands mit dem Ausgang des Funktionsverstärkers verbunden ist, wobei der Ausgang des Funktionsverstärkers ebenso angeschlossen ist, um den Oszillator anzutreiben.

17. Telemetriesystem nach Anspruch 9, zusätzlich umfassend:

einen Verstärker und Bandpaßfilter, der als einen Eingang den Ausgang von dem AM-Demodulator hat, wobei der Verstärker und Bandpaßfilter einen Ausgang liefern; und einen Dekodierer, der als einen Eingang den Ausgang von dem Verstärker und dem Bandpaßfilter hat.

18. Telemetriesystem nach Anspruch 9, zusätzlich umfassend:

einen FM-Demodulator, der parallel zu dem Induktor und dem Kondensator angeschlossen ist, wobei der FM-Demodulator einen Ausgang hat; einen Verstärker und Bandpaßfilter, die als einen Eingang den Ausgang von dem FM-Demodulator haben, wobei der Verstärker und Bandpaßfilter einen Ausgang liefern; und einen Dekodierer, der als einen Eingang den Ausgang von dem Verstärker und Bandpaßfilter hat.

19. Ein Verfahren zum Datentelemetrieren von Daten zwischen einer implantierbaren Vorrichtung und einer externen Vorrichtung, umfassend:

Senden eines Information enthaltenden magnetischen Feldes; Empfangen eines Information enthaltenden magnetischen Feldes mit einem Induktor; Verbinden eines Kondensators parallel zu dem Induktor; Antreiben des Induktors und des Kondensators mit einem Oszillator, der ein oszillierendes Ausgangssignal hat; Demodulieren des Signals parallel zu dem Induktor und dem Kondensator mit einem AM-Demodulator, wobei der AM-Demodulator einen Ausgang hat, welcher eine Wechselspannungskomponente und eine Gleichspannungskomponente enthält;

wobei das Verfahren **gekennzeichnet ist durch**

Aufrechterhalten der Gleichspannungskomponente des Ausgangs des AM-Demodulators auf konstantem Niveau durch Verbinden der Rückkopplungsschleifeneinrichtungen zwischen dem Ausgang des AM-Demodulators und dem Oszillator, um die Amplitude des oszillierenden Ausgangssignals zu steuern.

**Revendications**

1. Système de télémétrie destiné à être utilisé avec un dispositif implantable, comprenant :

une inductance destinée à recevoir un champ magnétique contenant des informations ; un condensateur connecté en parallèle aux bornes de l'inductance ; un oscillateur connecté de façon à exciter l'inductance et le condensateur avec un signal de sortie oscillant ; un démodulateur à modulation d'amplitude connecté aux bornes de l'inductance et du condensateur, le démodulateur à modulation d'amplitude ayant une sortie contenant une composante alternative et une composante continue ; le système étant caractérisé par: des moyens à boucle de rétroaction connectés entre la sortie du démodulateur à modulation d'amplitude et l'oscillateur pour commander l'amplitude du signal de sortie oscillant, le moyen à boucle de rétroaction étant destiné à maintenir constante la composante continue de la sortie du démodulateur à modulation d'amplitude.

2. Système de télémétrie suivant la revendication 1, dans lequel l'inductance et le condensateur ont des valeurs permettant de les accorder à une fréquence $f_0$, et l'oscillateur est réglé pour osciller à une fréquence qui est approximativement égale à $f_0$.

3. Système de télémétrie suivant la revendication 1, dans lequel l'oscillateur est commandé par les moyens à boucle de rétroaction pour maintenir à un niveau constant le signal excitant l'inductance et le condensateur.

4. Système de télémétrie selon la revendication 1, l'inductance ayant une première borne et une seconde borne, le condensateur ayant une première borne et une seconde borne, la première borne de l'inductance étant connectée à la première borne du condensateur, la seconde borne de l'inductance étant connectée à la seconde borne du condensateur, ces secondes bornes étant reliées à la terre, le démodulateur à modulation d'amplitude comprenant :

une diode ayant une cathode et une anode, l'anode étant connectée aux premières bornes de l'inductance et du condensateur ;

un second condensateur ayant des première et seconde bornes, la première borne du second condensateur étant connectée à la cathode de la diode, la seconde borne du second condensateur étant connectée à la seconde borne de l'inductance et à la seconde borne du condensateur ;

une résistance ayant des première et seconde bornes, la première borne de la résistance étant connectée à la première borne du second condensateur et à la cathode de la diode, la seconde borne de la résistance étant connectée à la seconde borne du second condensateur, à la seconde borne de l'inductance, et à la seconde borne du condensateur.

5. Système de télémétrie suivant la revendication 1, dans lequel les moyens à boucle de rétroaction sont conçus et configurés de façon à convertir la réponse du système d'une réponse passe-bas à un seul pôle en une réponse passe-bande.

6. Système de télémétrie suivant la revendication 1, dans lequel les moyens à boucle de rétroaction comprennent :

un amplificateur opérationnel ayant une entrée positive, une entrée négative et une sortie, l'entrée positive de l'amplificateur opérationnel recevant une tension de référence continue ;
une première résistance connectée entre la sortie du démodulateur à modulation d'amplitude et l'entrée négative de l'amplificateur opérationnel ;
un second condensateur ayant une première borne et une seconde borne, la première borne du second condensateur étant connectée à l'entrée négative de l'amplificateur opérationnel ; et
une seconde résistance ayant une première borne et une seconde borne, la première borne de la seconde résistance étant connectée à la seconde borne du second condensateur, la seconde borne de la seconde résistance étant connectée à la sortie de l'amplificateur opérationnel, la sortie de l'amplificateur opérationnel étant également connectée de façon à exciter l'oscillateur.

7. Système de télémétrie suivant la revendication 1, comprenant en outre :

un amplificateur et un filtre passe-bande ayant comme entrée la sortie du démodulateur à modulation d'amplitude, l'amplificateur et le filtre passe-bande fournissant une sortie ; et
un décodeur ayant comme entrée la sortie de l'amplificateur et du filtre passe-bande.

8. Système de télémétrie suivant la revendication 1, comprenant en outre :

un démodulateur à modulation de fréquence connecté aux bornes de l'inductance et du condensateur, le démodulateur à modulation de fréquence ayant une sortie ;
un amplificateur et un filtre passe-bande ayant comme entrée la sortie du démodulateur à modulation de fréquence, l'amplificateur et le filtre passe-bande fournissant une sortie ; et
un décodeur ayant comme entrée la sortie de l'amplificateur et du filtre passe-bande.

9. Système de télémétrie suivant l'une quelconque des revendications précédentes, comprenant en outre :

une autre inductance pour transmettre le champ magnétique contenant des informations ;
un autre condensateur connecté en parallèle aux bornes de la première inductance ;
un commutateur connecté en parallèle aux bornes de l'autre inductance et de l'autre condensateur ; et
des moyens pour ouvrir et fermer périodiquement et séquentiellement le commutateur afin de moduler une tension aux bornes de la première inductance et du premier condensateur.

10. Système de télémétrie suivant la revendication 9, dans lequel les moyens destinés à ouvrir et fermer périodiquement et séquentiellement le commutateur comprennent :

un codeur numérique, le commutateur étant ouvert lorsque la sortie du codeur numérique est un "0" numérique et étant fermé lorsque la sortie du codeur numérique est un "1" numérique.

11. Système de télémétrie suivant la revendication 10, dans lequel un bit de donnée de "0" est codé par le codeur numérique de façon à être constitué de deux excursions de zéro à un ou de un à zéro, tandis qu'un bit de donnée de "1" est codé par le codeur numérique de façon qu'il soit constitué d'une seule excursion de zéro à un ou de un à zéro.

12. Système de télémétrie suivant la revendication 9, dans lequel l'autre inductance et l'autre condensateur ont des valeurs telles qu'ils sont accordés à une fréquence $f_0$, et l'oscillateur est réglé de façon à osciller à une fréquence qui est proche de $f_0$.

13. Système de télémétrie suivant la revendication 12, dans lequel l'inductance et le condensateur ont des valeurs telles qu'ils sont accordés à la fréquence $f_0$.

**14.** Système de télémétrie suivant la revendication 9, dans lequel l'oscillateur est commandé par les moyens à boucle de rétroaction de facon à maintenir à un niveau constant le signal excitant l'inductance et le condensateur.

**15.** Système de télémétrie suivant la revendication 9, dans lequel les moyens à boucle de rétroaction sont conçus et configurés de façon à convertir la réponse du système d'une réponse passe-bas à un seul pôle en une réponse passe-bande.

**16.** Système de télémétrie selon la revendication 9, dans lequel les moyens à boucle de rétroaction comprennent :

un amplificateur opérationnel ayant une entrée positive, une entrée négative et une sortie, l'entrée positive de l'amplificateur opérationnel recevant une tension de référence continue ;
une première résistance connectée entre la sortie du démodulateur à modulation d'amplitude et l'entrée négative de l'amplificateur opérationnel ;
un second condensateur ayant une première borne et une seconde borne, la première borne du second condensateur étant connectée à l'entrée négative de l'amplificateur opérationnel ; et
une seconde résistance ayant une première borne et une seconde borne, la première borne de la seconde résistance étant connectée à la seconde borne du second condensateur, la seconde borne de la seconde résistance étant connectée à la sortie de l'amplificateur opérationnel, la sortie de l'amplificateur opérationnel étant également connectée de façon à exciter l'oscillateur.

**17.** Système de télémétrie selon la revendication 9, comprenant en outre :

un amplificateur et un filtre passe-bande ayant comme entrée la sortie du démodulateur à modulation d'amplitude, l'amplificateur et le filtre passe-bande fournissant une sortie ; et
un décodeur ayant comme entrée la sortie de l'amplificateur et du filtre passe-bande.

**18.** Système de télémétrie selon la revendication 9, comprenant en outre :

un démodulateur à modulation de fréquence connecté aux bornes de l'inductance et du condensateur, le démodulateur à modulation de fréquence ayant une sortie ;

un amplificateur et un filtre passe-bande ayant comme entrée la sortie du démodulateur à modulation de fréquence, l'amplificateur et le filtre passe-bande fournissant une sortie ; et
un décodeur ayant comme entrée la sortie de l'amplificateur et du filtre passe-bande.

**19.** Procédé de télémétrie de données entre un dispositif implantable et un dispositif externe, comprenant :

la transmission d'un champ magnétique contenant des informations ;
la réception d'un champ magnétique contenant des informations au moyen d'une inductance ;
la connexion d'un condensateur en parallèle aux bornes de l'inductance ;
l'excitation de l'inductance et du condensateur par un oscillateur ayant un signal de sortie oscillant ;
la démodulation du signal aux bornes de l'inductance et du condensateur par un démodulateur à modulation d'amplitude, ce démodulateur à modulation d'amplitude ayant une sortie contenant une composante alternative et une composante continue ; ce procédé étant caractérisé par :
le fait de maintenir constante la composante continue de la sortie du démodulateur à modulation d'amplitude en connectant des moyens à boucle de rétroaction entre la sortie du démodulateur à modulation d'amplitude et l'oscillateur afin de commander l'amplitude du signal de sortie oscillant.

*FIG. 1*

*FIG. 2*

*FIG. 3*

*FIG. 8*

NRZ
DATA

ENCODER
OUTPUT

SWITCH
STATE
CLOSED

OPEN

VOLTAGE ON
CAPACITOR *32*

OUTPUT OF
AM DEMODULATOR *40*

AMP & BANDPASS
FILTER *56* OUTPUT

DECODER
OUTPUT

*FIG.9*

$Fig. 5$

$Fig. 6$

$Fig. 7$

EP 0 516 617 B1

NRZ SERIAL
DATA (8192 BPS)

FEEDBACK
CIRCUIT — 54

Σ 52

+

− 

○ V_R

DIGITAL
ENCODER — 26

OSCILLATOR — 50

AM
DEMODULATOR — 40

V_O

AMPLIFIER
& BANDPASS
FILTER — 56

DECODER — 58

24  20  30  32  34  36

REFLECTED IMPEDANCE

NRZ SERIAL
DATA OUT (8192 BPS)

*Fig. 4*

---

NRZ SERIAL
DATA (8192 BPS)

FEEDBACK
LOOP — 54

Σ 52

+

−

○ V_R

DIGITAL
ENCODER — 26

OSCILLATOR — 50

AM
DEMODULATOR — 40

V_O

FM
DEMODULATOR — 80

AMPLIFIER
& BANDPASS
FILTER — 56

DECODER — 58

24  20  30  32  34  36

REFLECTED IMPEDANCE

*Fig. 10*

NRZ SERIAL
DATA OUT (8192 BPS)